# EUROPEAN PATENT APPLICATION

(11) **EP 4 218 747 A1**
(43) Date of publication of application: **02.08.2023**
(21) Application number: 21872491.2
(22) Date of filing: 22.09.2021
(51) Int. Cl.: A61K 31/135, A61K 9/20, A61K 9/24, A61P 25/04

(54) **FORMULATION FOR ORAL ADMINISTRATION**

(30) Priority: 25.09.2020 JP 2020160583
(71) Applicant: NIPPON ZOKI PHARMACEUTICAL CO., LTD., Chuo-ku Osaka-shi Osaka 541-0046 (JP)
(72) Inventor: ADACHI, Tsuyoshi, Osaka-shi, Osaka 541-0046 (JP); MITSUDA, Kenji, Kato-shi, Hyogo 673-1461 (JP); NAKANO, Hideshi, Osaka-shi, Osaka 541-0046 (JP)
(74) Representative: Hoffmann Eitle
(86) International application number: PCT/JP2021/034790
(87) International publication number: WO 2022/065362

(57) **Abstract**

The problem of the present invention is to provide an oral preparation that contains tramadol or a pharmaceutically acceptable salt thereof as an active ingredient and has a low incidence of side effects. The present invention provides an oral preparation such that the blood concentration of unchanged tramadol or an active metabolite thereof, for example, falls within a certain numerical range after administration, so that the incidence of side effects is low while the efficacy is excellent.

## Description

### [Technical Field]

The present invention relates to a preparation for oral administration containing tramadol or a pharmaceutically acceptable salt thereof as an active ingredient and having a low incidence of side effects. The present invention relates more specifically to a preparation for oral administration that the blood concentration of unchanged tramadol or an active metabolite thereof, for example, falls within a certain range at the time of oral administration, so that the incidence of side effects is low while the efficacy is excellent.

### [Background Art]

Tramadol [Chemical name: (1RS,2RS)-2-[(dimethylamino)methyl]-1-(3-methoxyphenyl)cyclohexanol] is a compound synthesized by Gruenthal GmbH in Germany in 1962. Tramadol has been marketed as an opioid analgesic in various dosage forms (e.g., oral agents, suppositories, injections, combination drugs) over 100 countries worldwide. This indicates that tramadol is a remarkable drug that has been supported by the market over many years. Regardless of the dosage form, the active ingredient of the tramadol preparation is usually tramadol hydrochloride.

Tramadol is a non-narcotic weak opioid classified as a second-step drug in the WHO guideline for cancer pain relief. In our country (Japan), a pharmacological effect for non-cancerous chronic pain on tramadol is also covered by insurance, and tramadol is used as a highly versatile analgesic. Tramadol itself only exerts weak noradrenaline/serotonin reuptake inhibitory activity, and is demethylated *in vivo* and metabolized into active metabolites (mainly M1 described below) exhibiting weak p-opioid receptor activity. The weak p-opioid receptor activity and the noradrenaline reuptake inhibitory activity act synergistically. Therefore, it is considered that tramadol elicits a favorable analgesic action and is effective for both nociceptive pain and neuropathic pain.

Tramadol is a synthetic compound which is phenol ethers having an amino group, and is a racemic mixture containing equivalent amounts of (+)-tramadol and (-)-tramadol enantiomers. Tramadol is water-soluble and is rapidly almost completely absorbed after oral administration. In the case of an oral capsule, the maximum drug concentration is reached 1.6 to 1.9 hours after administration, and the half-life is about 5 to 6 hours. On the other hand, the maximum drug concentration of O-desmethyltramadol (M1), an active metabolite, is reached by 2 hours after administration, and the half-life is 6 to 7 hours. The maximum concentration of orally administered tramadol in the brain is reached at 10 minutes, whereas the maximum concentration of the M1 metabolite is reached after 20 to 60 minutes. In addition, in the blood, the ratio of tramadol/M1 metabolite is from 0.5 to 1.0, indicating that the concentration of M1 metabolite is higher. On the other hand, the intracerebral migration of M1 metabolite is lower than that of tramadol. Therefore, tramadol is present at 4 to 15 times higher concentration than the M1 metabolite in the brain.

The metabolic pathway of tramadol is as follows: tramadol is subjected to O-demethylation mainly by cytochrome P450 (CYP) 2D6, and part thereof is subjected to N-demethylation by CYP3A4 (first phase reaction); and the demethylated products are then subjected to glucuronic acid conjugation or sulfuric acid conjugation (second phase reaction). Tramadol is O-demethylated to O-desmethyltramadol (M1), N-demethylated to N-desmethyltramadol (M2) and N,N-didesmethyltramadol (M3), or O-demethylated and N-demethylated in combination to O,N,N-tridesmethyltramadol (M4) or O,N-didesmethyltramadol (M5). M1, M4, and M5 are further subjected to glucuronic acid conjugation or sulfuric acid conjugation by a second phase reaction to form M6 to M11 conjugates. Among them, M1 and M1 conjugate, M5 and M5 conjugate, and M2 are major metabolites, and M3, M4, and M4 conjugate are minor. In addition, many other post-M12 metabolites have also been reported. Unchanged tramadol and metabolites thereof (about 90%) are mainly excreted through the kidney. Note that in the present application, the term "tramadol" refers to an unchanged drug unless otherwise indicated.

Previous studies have shown that the Ki value of (±)-tramadol for µ-opioid receptors is about from a few hundredths to a few thousandths of the affinity of morphine. Also, the affinity for δ- or κ-opioid receptor is even weaker. Therefore, it is considered unlikely that tramadol itself acts through the opioid receptors. Among the enantiomers of tramadol, the affinity of (+)-tramadol for the µ-opioid receptor is stronger than that of (-)-tramadol. In contrast, among tramadol metabolites, (±)-M1 has increased affinity for the opioid receptors, and in particular, (+)-M1 has high affinity. The following document has been published as a document summarizing the above findings (Table 1).

**[Table 1]**

| Binding affinity for opioid receptors and monoamine reuptake inhibitory activity of tramadol | | | | | |
|---|---|---|---|---|---|
| Test substance | Ki (µmol/L) | | | | |
| | *µ* | *δ* | *κ* | Noradrenaline | Serotonin |
| (±)-Tramadol | 2.12 | 57.7 | 42.7 | 0.785 | 0.992 |
| (+)-Tramadol | 1.33 | 6.24 | 54.0 | 2.51 | 0.528 |
| (-)-Tramadol | 24.8 | 213 | 53.5 | 0.432 | 2.35 |
| (±)-M1 | 0.0121 | 0.911 | 0.242 | 1.52 | 5.18 |
| (+)-M1 | 0.00602 | 0.594 | 0.706 | 14.4 | 2.98 |
| (-)-M1 | 0.428 | 30.5 | 32.9 | 0.857 | 17.7 |
| Codeine | 0.160 | 5.13 | 5.97 | NA | NA |
| Morphine | 0.000345 | 0.0925 | 0.662 | NA | NA |
| Imipramine | 3.73 | 12.7 | 1.82 | 0.00661 | 0.0211 |

| | | | | | |
|---|---|---|---|---|---|
| Mean value (n=2-3) NA : no activity at 10µmol/L (From "Tramcet (registered trademark) combination tablet information" website created by Mochida Pharmaceutical Co., Ltd.) | | | | | |

The above indicates that the effects of tramadol on µ-opioid receptors are mainly exerted not by tramadol itself, but M1 metabolites, particularly (+)-M1. However, when compared with other strong opioids such as morphine and fentanyl, the (+)-M1 metabolites are known to have binding affinity about a few tenths to a few hundredths of that in humans.

Tramadol, which exhibits the above pharmacokinetics and pharmacological actions, is known to cause main side effects such as constipation, nausea (retching), vomiting, somnolence, anorexia, floating dizziness, headache, thirst, and physical weariness. Tramadol, which is a kind of opioid, albeit a relatively weak one as described above, inevitably causes the side effects. However, it is considered that the frequency of side effects of tramadol is lower than that of each strong opioid such as morphine. In this regard, the side effects of opioids vary greatly among individuals. Tramadol also exerts noradrenaline/serotonin reuptake inhibitory activity as described above. Therefore, it should be noted that the side effects may be strengthened.

### [Summary of the Invention]

### [Problem to be Solved by the Invention]

The present inventors have found a surprising finding in the course of clinical development of a twice-daily oral tramadol preparation (tablet) (hereinafter, sometimes referred to as "the present preparation"), which has not been marketed in Japan. The present invention has then been completed.

That is, it has been found that the incidence of side effects is lower in the present preparation than in a four times daily oral preparation (capsule) or a once-daily oral preparation (tablet) (hereinafter, the former may be referred to as "other drug A"; the latter may be referred to as "other drug B"; and both may be collectively referred to as "other drugs"). Further, as a result of intensive analysis, the effect (hereinafter, sometimes referred to as "the present effect") that the incidence of side effects is lower in the present preparation than in other drugs seems to be due to a difference in pharmacokinetic parameters such as the blood concentration of unchanged tramadol or M1 metabolite after the present preparation or each other drug is administered.

### [Means for Solving the Problems]

The following (Table 2) shows the results of comparing the pharmacokinetic parameters of unchanged tramadol or M1 in the plasma of a subject when the present preparation containing 100 mg of tramadol hydrochloride was administered once to a healthy adult subject under fasted conditions with the pharmacokinetic parameters of other drugs, which have been found from published data.

**[Table 2]**

| Comparison of pharmacokinetic parameters between formulations at 100 mg oral single dose (mean ± standard deviation) | | | | |
|---|---|---|---|---|
| Pharmacokinetic parameters | | Present preparation ^{a)} n = 42 | Other drug B ^{b)} n=10 | Other drug A ^{c)}n=6 |
| Unchanged tramadol | Cₘₐₓ(ng/mL) | 213±41 | 123±39 | 342 ±73 |
| | tₘₐₓ(hr) | 1.2±0.6 | 9.5±2.8 | 1.5±0.8 |
| | t_{1/2}(hr) | 7.84±1.00 | 6.44±1.07 | 5.31±1.57 |
| | AUC_{0-inf}(ng•hr/mL) | 2428±791 | 2640±1020 | 2682±1182 |
| M1 | Cₘₐₓ(ng/mL) | 52.8±17.0 | 25.9±5.9 | 86.8±33.7 |
| | tₘₐₓ(hr) | 1.8±1.4 | 11.5±4.0 | 2.0±1.1 |
| | t_{1/2}(hr) | 9.68±1.56 | 7.02±1.37 | 6.09+1.69 |
| | AUC_{0-inf}(ng•hr/mL) | 842±197 | 610±159 | 808±184 |

| | | | | |
|---|---|---|---|---|
| a) Bioequivalence studies, Food effect study of Application formulation b) Drug Interview Form (Other drug B) c) Drug Interview Form (Other drug A) | | | | |

The results in Table 2 show that the maximum drug concentration (Cmax) of the unchanged compound in the present preparation is an intermediate value between those of the other drug A and the other drug B. On the other hand, it is characterized in that a time to maximum plasma concentration (Tmax) is shorter than that of the other drug A or the other drug B, and the plasma elimination half-life (T_{1/2}) is longer than that of the other drug A or the other drug B. There was no significant difference in the area under plasma concentration time curve from 0 hours to infinity (AUC₀₋inf).

Meanwhile, regarding the M1 metabolite, the maximum drug concentration (Cmax) of M1 in the present preparation was also an intermediate value between those of the other drug A and the other drug B. A time to maximum plasma concentration (Tmax) was shorter than that of the other drug A or other drug B. The plasma elimination half-life (T_{1/2}) was longer than that of the other drug A and the other drug B. These points were similar to those of the unchanged compound.

Next, the following shows the results of comparing the blood concentration after administration of a daily dose (100 mg × 2 times) of the present preparation with the blood concentration after administration of the other drug A (50 mg × 4 times) or the other drug B (200 mg × 1 time), which concentration has been found from publicly available data. (Table 3)

**[Table 3]**

| Comparison of blood concentrations after administration of a daily dose between formulations (mean ± standard deviation) | | | | |
|---|---|---|---|---|
| Pharmacokinetic parameters | | Twice daily dosing Present preparation 100mg×2 times n = 42 | Once daily dosing Other drug B ^{b)} 200mg×1 times n = 24 | Four times daily dosing Other drug Ab) 50mg×4 times n = 24 |
| Unchanged tramadol | Cₘₐₓ (ng/mL) | 280±60 | 283±66 | 308±67 |
| | AUC_{0·inf}(ng·hr/mL)^{a)} | 4868±1754 | 5880±1660 | 5810±1770 |
| Active metabolite M1 | Cₘₐₓ (ng/mL) | 76.9±21.8 | 59.8±23.0 | 63.6±21.8 |
| | AUC_{0·inf}(ng·hr/mL)^{a)} | 1684±395 | 1370±450 | 1370±400 |

| | | | | |
|---|---|---|---|---|
| a) Present preparation b) Drug Interview Form (Other drug B) | | | | |

The results in Table 3 have revealed that the maximum drug concentration (Cmax) of M1 metabolite in the present preparation at the time of daily dose administration is 21 to 29% higher than that of each other drug. It can also be seen that the area under plasma concentration time curve (AUC_{0-inf}) is 23% higher in the present preparation than in each other drug.

Incidentally, it has been found that, even when the present preparation or each other drug is repeatedly administered, substantially the same data as at the time of the daily dose administration can be obtained.

As described above, M1 metabolites intrinsically exerts µ-opioid receptor agonistic action. Thus, a higher concentration thereof should increase the frequency of incident of opioid-specific side effects. On the contrary, the outcome of the clinical trials conducted by the applicant was the opposite.

That is, surprisingly, the following shows the comparison between the incidence of side effects in the clinical trials for the present preparation and that for each other drug. Each incidence of side effects is collectively provided as those observed during the respective clinical trials. (Table 4)

**[Table 4]**

| Preparations | | Once daily dosage Other drug B | Four times daily dosage Other drug A | Twice daily dosage Present preparation |
|---|---|---|---|---|
| Pooled analysis target | | 3 studies | 5 studies | 6 studies |
| Number of evaluation cases | | 646 cases | 762 cases | 749 cases |
| Incidence of side effects | All | 91% | 85% | 80% |
| | Constipation | 62% | 51% | 41% |
| | Nausea | 52% | 49% | 44% |
| | Somnolence | 28% | 30% | 21% |
| | Vomiting | 23% | 19% | 15% |
| | Floating Dizziness | 18% | 19% | 11% |

The reason why the results as shown in Table 4 are obtained is unknown. Here, the above-mentioned side effects are side effects generally observed by use of opioids. Therefore, it is clear that the difference in the incidence of side effects between the present preparation and each other drug is not due to the difference in additives used in the production between the preparation and the drug. What is certain is that there is the fact that the low incidence of side effects was observed when the unchanged tramadol or the M1 metabolite exhibited pharmacokinetics (hereinafter, sometimes referred to as "the present pharmacokinetics") as described above in the case of single or multiple dosing.

Since the number of subjects (the number of cases) in each of the above clinical trials is large, the obtained data on the incidence of side effects is highly reliable.

The present inventors have arrived at the present invention on the basis of the above fact. Specifically, the present invention is as follows, but is not at all limited thereto.

(1) A preparation for oral administration containing tramadol or a pharmaceutically acceptable salt thereof as an active ingredient, wherein when an amount equivalent to 100 mg of the active ingredient is orally administered once to a human, a maximum drug concentration (Cmax) of the tramadol in the human is from 150 to 250 ng/mL.
(2) The preparation according to (1), wherein a time to maximum plasma concentration (Tmax) of the tramadol is from 1 to 1.4 hours.
(3) The preparation according to (1) or (2), wherein a plasma elimination half-life (T_{1/2}) of the tramadol is from 6.5 to 9.5 hours.
(4) The preparation according to any one of (1) to (3), wherein an area under plasma concentration time curve from 0 hours to infinity (AUC_{0-inf}) of the tramadol is from 1800 to 3000 ng hr/mL.
(5) A preparation for oral administration containing tramadol or a pharmaceutically acceptable salt thereof as an active ingredient, wherein when an amount equivalent to 100 mg of the active ingredient is orally administered once to a human, a maximum drug concentration (Cmax) of an active metabolite O-desmethyltramadol (M1) in the human is from 40 to 65 ng/mL.
(6) The preparation according to (5), wherein a time to maximum plasma concentration (Tmax) of the M1 is 1.5 hours or longer and less than 2 hours.
(7) The preparation according to (5) or (6), wherein a plasma elimination half-life (T_{1/2}) of the M1 is from 7.5 to 12 hours.
(8) The preparation according to any one of (5) to (7), wherein an area under plasma concentration time curve from 0 hours to infinity (AUC_{0-inf}) of the M1 is from 650 to 1000 ng hr/mL.
(9) The preparation according to any one of (1) to (8), wherein the active ingredient is tramadol hydrochloride.
(10) The preparation according to any one of (1) to (9), which is a twice daily administration type.
(11) The preparation according to (10), which is a tablet.
(12) The preparation according to (11), which is a bilayer tablet having an immediate-release part and a sustained-release part.
(13) The preparation according to any one of (1) to (12), wherein a dissolution rate of the active ingredient from the preparation is from 30 to 50 wt% after 15 minutes, from 40 to 60 wt% after 1 hour, from 50 to 70 wt% after 2 hours, from 60 to 80 wt% after 4 hours, and from 70 to 90 wt% after 6 hours in a dissolution test conducted at 50 rotations per minute by using 900 mL of a test liquid at a liquid temperature of 37°C while the dissolution test according to the second method (paddle method) of Dissolution test in General Tests in Japanese Pharmacopoeia.
(14) The preparation according to (13), wherein the dissolution rate of the active ingredient is from 35 to 45 wt% after 15 minutes, from 45 to 55 wt% after 1 hour, from 55 to 65 wt% after 2 hours, from 65 to 75 wt% after 4 hours, and from 75 to 85 wt% after 6 hours.
(15) A preparation for oral administration comprising tramadol or a pharmaceutically acceptable salt thereof as an active ingredient, wherein when an amount equivalent to 100 mg of the active ingredient is orally administered twice per day to a human, a maximum drug concentration (Cmax) of an active metabolite O-desmethyltramadol (M1) in the human is 65 ng/mL or higher.
(16) The preparation according to (15), wherein the Cmax of the M1 is from 65 to 85 ng/mL.
(17) The preparation according to (15) or (16), wherein an area under plasma concentration time curve from 0 hours to infinity (AUC_{0-inf}) of the M1 is from 1500 ng hr/mL or larger.
(18) The preparation according to any one of (15) to (17), wherein the AUC_{0-inf} of the M1 is from 1500 to 1750 ng hr/mL.
(19) The preparation according to any one of (15) to (18), wherein the active ingredient is tramadol hydrochloride.
(20) The preparation according to any one of (15) to (19), which is a twice daily administration type.
(21) The preparation according to (20), which is a tablet.
(22) The preparation according to (21), which is a bilayer tablet having an immediate- release part and a sustained-release part.
(23) The preparation according to any one of (15) to (22), wherein a dissolution rate of the active ingredient from the preparation is from 30 to 50 wt% after 15 minutes, from 40 to 60 wt% after 1 hour, from 50 to 70 wt% after 2 hours, from 60 to 80 wt% after 4 hours, and from 70 to 90 wt% after 6 hours in a dissolution test conducted at 50 rotations per minute by using 900 mL of a test liquid at a liquid temperature of 37°C while the dissolution test according to second method (paddle method) of Dissolution test in General Tests in Japanese Pharmacopoeia.
(24) The preparation according to (23), wherein the dissolution rate of the active ingredient is from 35 to 45 wt% after 15 minutes, from 45 to 55 wt% after 1 hour, from 55 to 65 wt% after 2 hours, from 65 to 75 wt% after 4 hours, and from 75 to 85 wt% after 6 hours.

### [Advantages of the Invention]

The preparation according to the present invention (the present preparation) has an effect (the present effect) such that the side effects are less than those of conventional tramadol-containing oral preparations.

The present effect is not an effect exhibited based on what kind of additive was blended with an active ingredient tramadol in what kind of ratio. Any formulation of a tramadol-containing oral preparation exhibiting substantially the same pharmacokinetics, such as a blood concentration, as of the above-described present preparation should exert substantially the same effects. In this sense, the formulation of the present invention is not at all limited as long as the present preparation is an oral preparation containing tramadol as an active ingredient.

### [Mode for Carrying Out the Invention]

As described above, the preparation according to the present invention (the present preparation) is not limited to a specific formulation.

However, an example of the formulation of the present preparation is as follows. Specifically, examples of the formulation of the present preparation include the formulation of Example 1 disclosed in the published international application (International Publication No. WO 2007/114376). When a preparation for oral administration disclosed as the Example 1 is produced, the preparation has the characteristics described in the international application and exhibits the present pharmacokinetics. However, the present invention is not at all limited thereto.

Meanwhile, the preparation for oral administration may be, for example, a tablet. In this case, when the one tablet contains 100 mg of tramadol or a pharmaceutically acceptable salt thereof and exhibits the present pharmacokinetics, the tablet naturally corresponds to the present preparation. In addition, one tablet may contain, for example, 50 mg of tramadol or a pharmaceutically acceptable salt thereof and may exhibit the present pharmacokinetics when the two tablets are administered. In this case, the tablet also corresponds to the present preparation. As used herein, the expression "amount equivalent to 100 mg" is used in such a sense.

As repeatedly mentioned, the present preparation is characterized by a low incidence of opioid-specific side effects.

However, as a matter of course, the results of the clinical trials conducted by the present applicant have proved the efficacy of the present preparation, that is, an excellent analgesic effect of the present preparation.

The following shows the results of the clinical trials on the efficacy of the present preparation.

### 1. Results of phase III clinical trial on knee osteoarthritis in Japan

Each subject enrolled was a patient with chronic pain who had been diagnosed as knee osteoarthritis and received oral administration of a nonsteroidal anti-inflammatory analgesic (NSAIDs), which administration did not result in a sufficient analgesic effect. Here, 159 patients, who received a twice-daily dose over 1 to 4 weeks, which dose was appropriately increased in the range of 100 to 300 mg/day, in an open-label manner and who met the transition criteria to a double-blind phase, were randomly divided into 78 subjects of the present preparation group and 81 subjects of the placebo group. The present preparation or placebo, respectively, was administered for 4 weeks. The period was set from the start of the double-blind phase until the analgesic effect of the investigational drug became insufficient and the values of analgesic effect of each group were compared by a log-rank test. As a result, there was a significant difference in the present preparation group relative to the placebo group (p = 0.042), and the superiority of the present preparation group over the placebo group was verified.

### 2. Results of phase III clinical trial on post-herpetic neuralgia in Japan

Each subject enrolled was a patient with chronic pain who had been diagnosed as post-herpetic neuralgia and received oral administration of an analgesic adjuvant and/or a non-opioid analgesic, which administration did not result in a sufficient analgesic effect. Here, 171 patients, who received a twice-daily dose of the present preparation over 1 to 5 weeks, which dose was appropriately increased in the range of 100 to 400 mg/day, in an open-label manner and who met the transition criteria (note 1) to a double-blind phase, were randomly divided into 83 subjects of the present preparation group and 88 subjects of the placebo group. The present preparation or placebo, respectively, was administered for 4 weeks. The period was set from the start of the double-blind phase until the analgesic effect of the investigational drug became insufficient (note 2) and the values of analgesic effect of each group were compared by a log-rank test. As a result, there was a significant difference in the present preparation group relative to the placebo group (p = 0.0005), and the superiority of the present preparation group over the placebo group was verified.
(Note 1) After 1 week from the administration in the open-label period (increasing dose period), the case of satisfying the following criteria is subject to transition to the open-label period (dose-fixed period).When the criteria were unmet, the administration was discontinued.
   - The average of the longest continuous pain NRS (Numerical Rating Scale) value in the previous 3 days before each evaluation time point is improved by 2 or more from the average of the longest continuous pain NRS value in the previous 3 days before the start of the open-label period.
(Note 2) When any of the following criteria was satisfied during the double-blind phase, it was determined that the analgesic effect was insufficient, and the administration was discontinued.
   - 2 consecutive days in which the longest continuous pain NRS value in the past 24 hours during the double-blind phase is worsened by 2 or more than the average of the longest continuous pain NRS value in the past 24 hours in the previous 3 days before the start of the double-blind phase.
   - A subject requests to discontinue the administration of the investigational drug due to an insufficient analgesic effect.

### 3. Results of long-term dosing study on chronic pain

The subjects enrolled were 174 patients who had chronic pain due to any of low back pain, knee osteoarthritis, rheumatoid arthritis, spinal stenosis, post-herpetic neuralgia, painful diabetic neuropathy, fibromyalgia, or complex local pain syndrome and who had received oral administration of an analgesic adjuvant and/or a non-opioid analgesic, which administration did not result in a sufficient analgesic effect. The present preparation was administered twice a day in the range of 100 to 400 mg/day for 8 weeks, while the dose was appropriately changed, up to 52 weeks (12 months). The time-course of the average of the NRS value (maximum pain over the past 24 hours), which was graded in a 11 point scale of 0 to 10, was as follows. The average decreased over time throughout the dose adjustment period and continued to decrease until the 32nd week of the continuous dosing period; and the improved conditions were then maintained. The average (standard deviation) of the amount of change in the NRS value (maximum pain over the past 24 hours) was -3.18 (± 2.26) at the end of the dose adjustment period and remained between -3.20 and -3.83 in the continuous dosing period. In addition, the average (standard deviation) of the amount of change in the NRS value (the longest lasting pain over the past 24 hours) was -2.77 (± 2.08) at the end of the dose adjustment period. There was no major difference in the amount of change in each NRS value at the end of the dose adjustment period among the primary diseases. In all of the primary diseases, with a few exceptions, the improvement by 2 or more, which is considered clinically meaningful amount of change (Pain, 2001; 94 : 149-58; J Pain Symptom Manage, 2012, 44:340-50), was observed. (Table 5)

**[Table 5]**

| Change from baseline of each NRS value at final evaluation in each primary disease | | | |
|---|---|---|---|
| Primary disease | Number of cases | Maximum pain over past 24 hours | Longest lasting pain over past 24 hours |
| Low back pain | 29 cases | -3.13 | -2.88 |
| | | ±2.03 | ±2.07 |
| Knee osteoarthritis | 29 cases | -2.56 | -2.27 |
| | | ±2.09 | ±1.97 |
| Rheumatoid arthritis | 10 cases | -2.91 | -2.26 |
| | | ±2.45 | ±2.23 |
| Spinal stenosis | 25 cases | -2.76 | -2.38 |
| | | ±2.41 | ±2.02 |
| Post-herpetic neuralgia | 19 cases | -2.84 | -2.04 |
| | | ±2.32 | ±1.83 |
| Painful diabetic neuropathy | 17 cases | -3.64 | -3.39 |
| | | ±2.37 | ±2.31 |
| Fibromyalgia | 30 cases | -4.70 | -4.06 |
| | | ±1.98 | ±1.89 |
| Complex local pain syndrome | 11 cases | -1.95 | -1.85 |
| | | ±1.59 | ±1.49 |
| Total | 170 cases | -3.18 | -2.77 |
| | | ±2.26 | ±2.08 |

| | | | |
|---|---|---|---|
| (mean ± standard deviation) | | | |

### [Industrial Applicability]

As described above, the present invention relates to a preparation for oral administration that contains tramadol or a pharmaceutically acceptable salt thereof as an active ingredient and has a relatively low incidence of side effects while retaining excellent efficacy, and is thus highly useful.

## Claims

1. A preparation for oral administration containing tramadol or a pharmaceutically acceptable salt thereof as an active ingredient, wherein when an amount equivalent to 100 mg of the active ingredient is orally administered once to a human, a maximum drug concentration (Cmax) of the tramadol in the human is from 150 to 250 ng/mL.

2. The preparation according to claim 1, wherein a time to reach a maximum plasma concentration (Tmax) of the tramadol is from 1 to 1.4 hours.

3. The preparation according to claim 1 or 2, wherein a plasma elimination half-life (T_{1/2}) of the tramadol is from 6.5 to 9.5 hours.

4. The preparation according to any one of claims 1 to 3, wherein an area under plasma concentration time curve from 0 hours to infinity (AUC_{0-inf}) of the tramadol is from 1800 to 3000 ng·hr/mL.

5. A preparation for oral administration comprising tramadol or a pharmaceutically acceptable salt thereof as an active ingredient, wherein when an amount equivalent to 100 mg of the active ingredient is orally administered once to a human, a maximum drug concentration (Cmax) of an active metabolite O-desmethyltramadol (M1) in the human is from 40 to 65 ng/mL.

6. The preparation according to claim 5, wherein a time to maximum plasma concentration (Tmax) of the M1 is 1.5 hours or longer and less than 2 hours.

7. The preparation according to claim 5 or 6, wherein a plasma elimination half-life (T_{1/2}) of the M1 is from 7.5 to 12 hours.

8. The preparation according to any one of claims 5 to 7, wherein an area under plasma concentration time curve from 0 hours to infinity (AUC_{0-inf}) of the M1 is from 650 to 1000 ng hr/mL.

9. The preparation according to any one of claims 1 to 8, wherein the active ingredient is tramadol hydrochloride.

10. The preparation according to any one of claims 1 to 9, which is a twice daily administration type.

11. The preparation according to claim 10, which is a tablet.

12. The preparation according to claim 11, which is a bilayer tablet having an immediate-release part and a sustained release part.

13. The preparation according to any one of claims 1 to 12, wherein a dissolution rate of the active ingredient from the preparation is from 30 to 50 wt% after 15 minutes, from 40 to 60 wt% after 1 hour, from 50 to 70 wt% after 2 hours, from 60 to 80 wt% after 4 hours, and from 70 to 90 wt% after 6 hours in a dissolution test conducted at 50 rotations per minute by using 900 mL of a test liquid at a liquid temperature of 37°C while the dissolution test according to the second method (paddle method) of Dissolution test in General Tests in Japanese Pharmacopoeia.

14. The preparation according to claim 13, wherein the dissolution rate of the active ingredient is from 35 to 45 wt% after 15 minutes, from 45 to 55 wt% after 1 hour, from 55 to 65 wt% after 2 hours, from 65 to 75 wt% after 4 hours, and from 75 to 85 wt% after 6 hours.

15. A preparation for oral administration comprising tramadol or a pharmaceutically acceptable salt thereof as an active ingredient, wherein when an amount equivalent to 100 mg of the active ingredient is orally administered twice per day to a human, a maximum drug concentration (Cmax) of an active metabolite O-desmethyltramadol (M1) in the human is 65 ng/mL or higher.

16. The preparation according to claim 15, wherein the Cmax of the M1 is from 65 to 85 ng/mL.

17. The preparation according to claim 15 or 16, wherein an area under plasma concentration time curve from 0 hours to infinity (AUC_{0-inf}) of the M1 is from 1500 ng hr/mL or larger.

18. The preparation according to any one of claims 15 to 17, wherein the AUC_{0-inf} of the M1 is from 1500 to 1750 ng hr/mL.

19. The preparation according to any one of claims 15 to 18, wherein the active ingredient is tramadol hydrochloride.

20. The preparation according to any one of claims 15 to 19, which is a twice daily administration type.

21. The preparation according to claim 20, which is a tablet.

22. The preparation according to claim 21, which is a bilayer tablet having an immediate-release part and a sustained release part.

23. The preparation according to any one of claims 15 to 22, wherein a dissolution rate of the active ingredient from the preparation is from 30 to 50 wt% after 15 minutes, from 40 to 60 wt% after 1 hour, from 50 to 70 wt% after 2 hours, from 60 to 80 wt% after 4 hours, and from 70 to 90 wt% after 6 hours in a dissolution test conducted at 50 rotations per minute by using 900 mL of a test liquid at a liquid temperature of 37°C while the dissolution test according to the second method (paddle method) of Dissolution test in General Tests in Japanese Pharmacopoeia.

24. The preparation according to claim 23, wherein the dissolution rate of the active ingredient is from 35 to 45 wt% after 15 minutes, from 45 to 55 wt% after 1 hour, from 55 to 65 wt% after 2 hours, from 65 to 75 wt% after 4 hours, and from 75 to 85 wt% after 6 hours.
